(19) **Europäisches Patentamt / European Patent Office / Office européen des brevets**

(11) **EP 3 111 841 B1**

(12) **EUROPEAN PATENT SPECIFICATION**

(45) Date of publication and mention
of the grant of the patent:
**06.06.2018 Bulletin 2018/23**

(51) Int Cl.:
*A61B 5/11* (2006.01)   *A61B 5/00* (2006.01)
*A01K 29/00* (2006.01)

(21) Application number: **16169871.7**

(22) Date of filing: **17.05.2016**

(54) **EVALUATION DEVICE AND EVALUATION PROGRAM**

AUSWERTUNGSVORRICHTUNG UND AUSWERTUNGSPROGRAMM

DISPOSITIF D'ÉVALUATION ET PROGRAMME D'ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2015 JP 2015131987**

(43) Date of publication of application:
**04.01.2017 Bulletin 2017/01**

(73) Proprietor: **FUJITSU LIMITED
211-8588 Kanagawa (JP)**

(72) Inventor: **Yamamoto, Kiyoko
Osaka, 540-0001 (JP)**

(74) Representative: **Hoffmann Eitle
Patent- und Rechtsanwälte PartmbB
Arabellastraße 30
81925 München (DE)**

(56) References cited:
**WO-A2-2008/011590     CN-A- 104 523 282
JP-A- 2006 218 122     JP-A- 2015 084 943
US-A1- 2007 000 216**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

The embodiments discussed herein are related to an evaluation device, and an evaluation program.

BACKGROUND

General horses represented by a thoroughbred have a problem in the health management, which will be described below. In general, to improve the ability of a horse, a training for applying load on the mind and body of the horse is performed. However, the horse is a living being and a physical condition changes day by day. One abnormal change in the physical condition of the horse is an abnormal gait state called lameness. It is known that the lameness occurs due to the occurrence of any abnormality in an extremity of the horse. However, it may be difficult to detect lameness if the lameness is slight. Even when the lameness is slight, if a hard training is imposed on the horse with the lameness, a serious damage, such as a bowed tendon, may occur. Therefore, it is desirable to detect the lameness at an early stage, that is, while the lameness is slight, and take measures, such as reduction of the training or provision of medical treatment, in terms of the health management of the horse.

Patent Literature 1: International Publication Pamphlet No. 2005/115242
Patent Literature 2: International Publication Pamphlet No. 2006/009959
Patent Literature 3: Japanese Laid-open Patent Publication No. 2008-264114

Document JP 2006 218122 A describes a system comprising a comparison between a milk cow to diagnose and a health cow (normal cow) in respect of magnitude and direction of accelerations with which their respective body axes move, allowing objective and simple determination of the occurrence of a disease in any leg of the milk cow to diagnose and specification of a leg suffering from the disease. Further, the system comprises a comparison between the milk cow to diagnose after the leg suffering from the disease is cured and the same milk cow before cured in respect of magnitude and direction of accelerations with which their respective body axes move, allowing objective and simple evaluation of the stage after the leg suffering from the disease is cured.

Conventionally, lameness is detected by a person who manages a horse, on the basis of his/her experience and intuition. However, it may be difficult to discriminate slight lameness from a healthy state as described above. Furthermore, if the person who manages a horse does not have enough experience, he/she may overlook lameness that may be detected by a veteran. Moreover, the person who manages a horse usually manages a plurality of horses at one time, and therefore may overlook a slight abnormal change of each of the horses. Due to the above-described circumstances, slight lameness at an early stage may be overlooked and an inappropriate training may be imposed on the horse, so that a more serious damage may be induced.

Incidentally, while the lameness of a horse has been described above, the lameness occurs due to the occurrence of any abnormality in an extremity, so that the lameness can occur in general animals that move with four legs, such as cows and dogs.

According to an aspect, an object is to provide an evaluation device, and an evaluation program capable of easily detecting lameness. the above object is achieved by the subject matter of the independent claims where dependent claims describe advantageous embodiments.

According to an example, an evaluation device includes an evaluating unit that evaluates balance between right and left in movement, for each stride on the basis of measurement data obtained by a sensor worn by an animal that moves with four legs; and an output unit that outputs evaluation results of a plurality of strides obtained by the evaluating unit.

BRIEF DESCRIPTION OF DRAWINGS

FIG. 1 is a diagram illustrating an example of an overall configuration of a system;
FIG. 2 is a diagram illustrating an example of the flow of managing the health of a horse by the system according to a first embodiment;
FIG. 3 is a diagram illustrating an example of a functional configuration of a measuring device according to the first embodiment;
FIG. 4 is a diagram illustrating an example of a functional configuration of an evaluation device according to the first embodiment;
FIG. 5 is a diagram illustrating an example of a trajectory of positions;

FIG. 6 is a diagram illustrating an example of a trajectory of the position of the brisket of a horse on a plane;

FIG. 7 is a diagram illustrating an example of a trajectory of the position of the brisket of a horse;

FIG. 8 is a diagram illustrating an example of a left area and a right area of the trajectory with respect to a center line CL;

FIG. 9 is a diagram illustrating an example of left portions and right portions of a line segment L1 and a line segment L2 with respect to the center line CL;

FIG. 10 is a diagram illustrating an example of reduction amounts of a point P1 and a point P3 from an intersection CP;

FIG. 11A is a diagram illustrating an example in which balance between right and left is not appropriately evaluated;

FIG. 11B is a diagram illustrating an example in which the balance between right and left is not appropriately evaluated;

FIG. 11C is a diagram illustrating an example in which the balance between right and left is not appropriately evaluated;

FIG. 12A is a diagram illustrating an example of a distribution ratio of the balance between right and left;

FIG. 12B is a diagram illustrating examples of a distribution ratio of the balance between right and left;

FIG. 13 is a diagram illustrating an example of a transition of an evaluation result of the balance between right and left for each of a plurality of strides;

FIG. 14 is a flowchart illustrating an example of the flow of an evaluation process;

FIG. 15 is a diagram illustrating an example of a functional configuration of an evaluation device according to a second embodiment;

FIG. 16 is a diagram illustrating an example of the flow of managing the health of a horse by a system according to the second embodiment;

FIG. 17 is a diagram illustrating an example of the flow of managing the health of a horse by a system according to a third embodiment;

FIG. 18 is a diagram schematically illustrating inclination of a trajectory of a circular movement; and

FIG. 19 is a diagram illustrating an example of a configuration of a computer that executes an evaluation program.

## DESCRIPTION OF EMBODIMENT(S)

Preferred embodiments of the present invention will be explained with reference to accompanying drawings. The disclosed technology is not limited by the embodiments. Furthermore, the embodiments described below may be combined appropriately as long as no contradiction is derived.

[a] First Embodiment

System Configuration

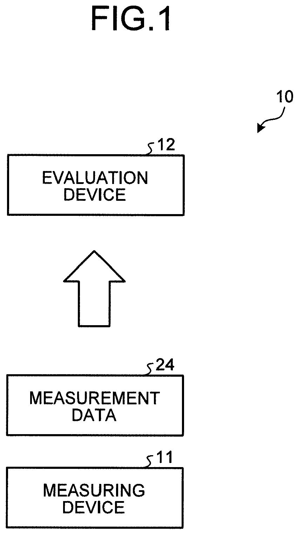

First, an example of a system that performs health management according to a first embodiment will be described. FIG. 1 is a diagram illustrating an example of an overall configuration of the system. A system 10 is a system that detects lameness of an animal, such as a horse, a cow, or a dog, that moves with four legs. In the following, an example will be described in which lameness of a horse as the animal that moves with four legs is detected.

To improve the ability of a horse, a training for applying load on the mind and body of the horse is performed. In particular, it is important to train a race horse, such as a thoroughbred, while appropriately managing a physical condition and preventing a physical damage. Therefore, lameness of the race horse needs to be detected at an early stage, that is, while the lameness is slight. In the first embodiment, the system 10 assists detection of the lameness of the horse.

As illustrated in FIG. 1, the system 10 includes a measuring device 11 and an evaluation device 12. The measuring device 11 is a device that is worn by a horse and measures a behavior of the horse when the horse moves with four legs. For example, the measuring device 11 includes various built-in sensors, and the various sensors measure the behavior of the horse when the horse moves with four legs. The measuring device 11 stores therein measurement data 24 measured by the various sensors. The measuring device 11 and the evaluation device 12 can transmit and receive data to and from each other via wire communication, wireless communication, or a storage medium, such as a flash memory. The measurement data 24 measured by the measuring device 11 is sent to the evaluation device 12 via wire communication, wireless communication, or a storage medium.

The evaluation device 12 is a device that evaluates balance between right and left when the horse moves with four legs, on the basis of the measurement data 24. The evaluation device 12 is, for example, a computer, such as a personal computer or a server computer. The evaluation device 12 may be, for example, a mobile terminal, such as a tablet terminal, a smartphone, or a personal digital assistant (PDA). For example, the evaluation device 12 is installed in a management source, such as a stable or a ranch, that manages the horse. The evaluation device 12 may be implemented as a single computer or may be implemented by a plurality of computers. In the first embodiment, an example will be described in which the evaluation device 12 is implemented as a single computer.

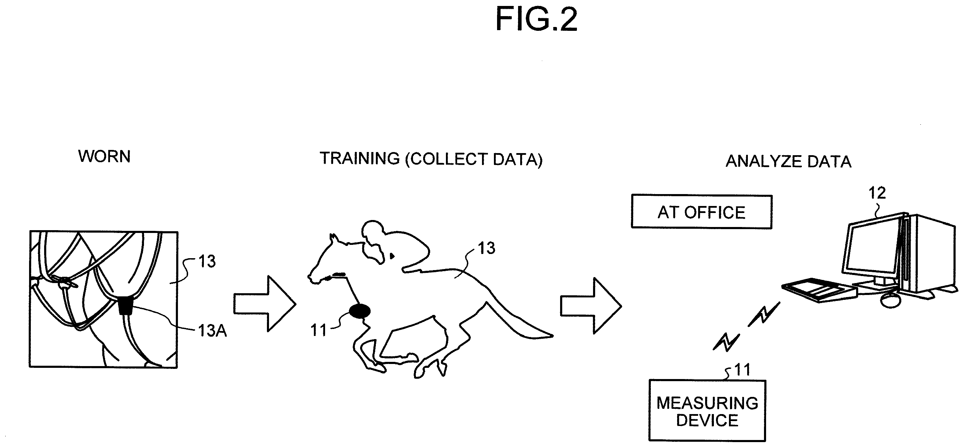

An example of the flow of managing the health of a horse by using the system 10 will be described. FIG. 2 is a diagram illustrating an example of the flow of managing the health of a horse by the system according to the first embodiment. The measuring device 11 is put on the brisket of a horse 13 to be subjected to health management. For example, the measuring device 11 is housed in a harness 13A worn by the horse 13. In the example in FIG. 2, the measuring device 11 is put on the brisket of the horse 13 by using a martingale. The horse 13 performs various trainings while wearing the measuring device 11. The measuring device 11 collects, by the various sensors, pieces of data on behaviors during the trainings, and stores therein the measurement data 24.

Incidentally, lameness occurs due to the occurrence of any abnormality in an extremity of a horse, and is, what is called, a state of dragging a leg. The horse with the lameness favors a leg in which the lameness has occurred, and therefore tends to lose the balance between right and left. To detect the balance between right and left of the horse, it may be possible to put a sensor on a leg of the horse. However, if the sensor is put on the leg of the horse, the weight of the sensor or the like may be a burden on the leg, and a physical damage may be induced. Furthermore, it may be possible to put the sensor on the head of the horse. However, the head is greatly influenced by movement of the neck, such as shaking of the neck, which makes it difficult to detect the balance between right and left of the horse. In contrast, the breast of the horse moves along with the forelegs, so that it is easy to detect the balance between right and left of the horse. Therefore, in the system 10 according to the first embodiment, the measuring device 11 is put on the brisket of the horse 13 and measures movement of the breast of the horse 13.

After the trainings, the measuring device 11 is brought to an office of the management source, and the stored measurement data 24 is uploaded on the evaluation device 12 via a storage medium or through wire communication or wireless communication. The evaluation device 12 evaluates the balance between right and left in movement, for each stride on the basis of the uploaded measurement data 24, and outputs an evaluation result.

Configuration of Measuring Device

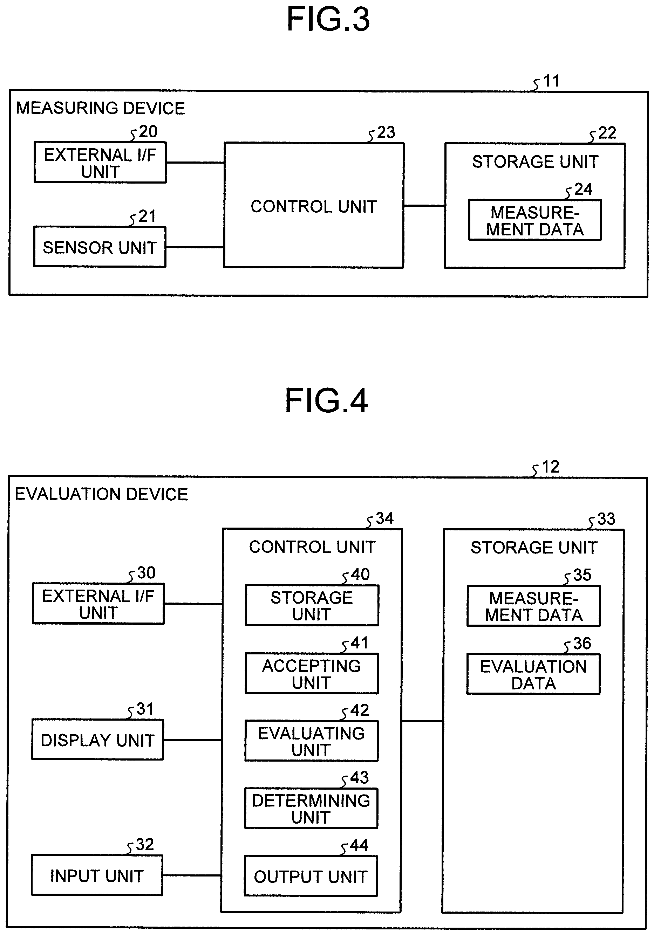

Configurations of the devices will be described below. First, a configuration of the measuring device 11 will be described. FIG. 3 is a diagram illustrating an example of a functional configuration of the measuring device according to the first embodiment. As illustrated in FIG. 3, the measuring device 11 includes an external interface (I/F) unit 20, a sensor unit 21, a storage unit 22, and a control unit 23.

The external I/F unit 20 is, for example, an interface that transmits and receives various kinds of information to and from other devices. In the measuring device 11 according to the first embodiment, the external I/F unit 20 is a port for inputting and outputting data to and from a storage medium, such as a flash memory, a communication port for performing wire communication via a cable or the like, or a communication interface for performing wireless communication.

The sensor unit 21 is a sensor that measures an acceleration and an angular velocity. For example, the sensor unit 21 is a six-axis sensor that measures accelerations in three-axis directions perpendicular to one another and measures angular velocities of the same three axes. Incidentally, the sensor unit 21 may include a three-axis acceleration sensor that measures accelerations in the three-axis directions and a gyro sensor that measures angular velocities of the three axes.

The storage unit 22 is a data rewritable semiconductor memory, such as a random access memory (RAM), a flash memory, or a non-volatile static random access memory (NVSRAM). The storage unit 22 may be a storage device, such as a hard disk, a solid state drive (SSD), or an optical disk. The storage unit 22 stores therein an operating system (OS) or various programs that are executed by the control unit 23. The storage unit 22 also stores therein various kinds of information. For example, the storage unit 22 stores therein the measurement data 24.

The measurement data 24 is data in which various kinds of information on behaviors of a horse are stored. For example, in the measurement data 24, values of the accelerations in the three-axis directions and the angular velocities of the three axes measured by the sensor unit 21 are stored in association with a measurement time.

The control unit 23 is a device that controls the entire measuring device 11. As the control unit 23, an electronic circuit, such as a central processing unit (CPU) or a micro processing unit (MPU), or an integrated circuit, such as an application specific integrated circuit (ASIC) or a field programmable gate array (FPGA), may be used.

The control unit 23 stores various kinds of data detected by the sensor unit 21 in the measurement data 24. For example, the control unit 23 measures the accelerations in the three-axis directions and the angular velocities of the three axes by using the sensor unit 21, at a predetermined period. At every measurement, the control unit 23 stores the values of the accelerations in the three-axis directions, the values of the angular velocities of the three axes, and the measurement time in the measurement data 24 in an associated manner. As the measurement time, it may be possible to use an elapsed time since a time of a starting point at which the measurement is started, or a global time that is measured by using a timestamp or the like. If the elapsed time is used as the measurement time, the measurement data 24 with a header, in which a measurement start date and time at which the measurement is started is embedded, is stored in the storage unit 22. In the following, it is assumed that the accelerations in the three-axis directions and the angular velocities of the three axes are measured at a period of 0.05 second; however, the measurement period is not limited to this example.

Configuration of Evaluation device

A configuration of the evaluation device 12 will be described below. FIG. 4 is a diagram illustrating an example of a functional configuration of the evaluation device according to the first embodiment. As illustrated in FIG. 4, the evaluation device 12 includes an external I/F unit 30, a display unit 31, an input unit 32, a storage unit 33, and a control unit 34.

The external I/F unit 30 is, for example, an interface that transmits and receives various kinds of information to and from other devices. In the evaluation device 12 according to the first embodiment, the external I/F unit 30 is a port for inputting and outputting data to and from a storage medium, such as a flash memory, a communication port for performing wire communication via a cable or the like, or a communication interface for performing wireless communication. For example, the external I/F unit 30 receives the measurement data 24 from the measuring device 11 through a storage medium, wire communication, or wireless communication.

The display unit 31 is a display device that displays various kinds of information. As the display unit 31, a display device, such as a liquid crystal display (LCD) or a cathode ray tube (CRT), may be used. The display unit 31 displays various kinds of information. For example, the display unit 31 displays various screens, such as an operation screen.

The input unit 32 is an input device for inputting various kinds of information. As the input unit 32, an input device, such as a mouse or a keyboard, that accepts an input of operations may be used. The input unit 32 may be various buttons provided on the evaluation device 12, or a transmissive touch sensor provided on the display unit 31. The input unit 32 accepts an input of various kinds of information. For example, the input unit 32 accepts an input of various operations related to evaluation, such as an instruction to start evaluation. The input unit 32 accepts an input of operations from a user, and inputs operation information indicating contents of the accepted operations to the control unit 34. In the example in FIG. 4, the display unit 31 and the input unit 32 are separated for the sake of illustration of the functional configuration. However, for example, it may be possible to construct a device, such as a touch panel, in which the display unit 31 and the input unit 32 are integrated.

The storage unit 33 is a storage device that stores therein various kinds of data. For example, the storage unit 33 is a storage device, such as a hard disk, an SSD, or an optical disk. The storage unit 33 may be a data rewritable semiconductor memory, such as a RAM, a flash memory, or an NVSRAM.

The storage unit 33 stores therein an OS or various programs that are executed by the control unit 34. The storage unit 33 also stores therein various kinds of information. For example, the storage unit 33 stores therein measurement data 35 and evaluation data 36.

The measurement data 35 is data in which the measurement data 24 acquired from the measuring device 11 is stored. The evaluation data 36 is data in which an evaluation result of the measurement data 35 is stored.

The control unit 34 is a device that controls the entire evaluation device 12. As the control unit 34, an electronic circuit, such as a CPU or an MPU, or an integrated circuit, such as an ASIC or an FPGA, may be used. The control unit 34 includes an internal memory for storing a program that defines the procedure of various processes and for storing control data, and performs various processes by using the program and the data. The control unit 34 functions as various processing units when various programs are operated. For example, the control unit 34 includes a storage unit 40, an accepting unit 41, an evaluating unit 42, a determining unit 43, and an output unit 44.

The storage unit 40 stores therein various kinds of data. For example, the storage unit 40 stores, as the measurement data 35 in the storage unit 33, the measurement data 24 that is acquired from the measuring device 11 through the external I/F unit 30.

The accepting unit 41 accepts various inputs. For example, the accepting unit 41 accepts various operation instructions. For example, the accepting unit 41 displays various screens, such as an operation screen, on the display unit 31, and accepts operation instructions, such as an instruction to start evaluation of the measurement data 35, from the input unit 32.

The evaluating unit 42 performs various evaluations. For example, the evaluating unit 42 evaluates the balance between right and left in movement, for each stride on the basis of the measurement data 35. An evaluation method will be described in detail below.

First, the evaluating unit 42 obtains a trajectory of the position of the brisket of a horse for each stride, on the basis of the measurement data 35. Incidentally, the accelerations in the three-axis directions at each measurement time stored in the measurement data 35 include oscillating components due to limb movement of the horse and gravity components due to the gravity. The oscillating components correspond to one stride of the horse and periodically change. In contrast, the gravity components are approximately constant. Therefore, by applying a low-pass filter (LPF) or a high-pass filter (HPF) with an appropriate cut-off frequency in accordance with changes in the accelerations in the three-axis directions, it is possible to separate the oscillating components of the horse and the gravity components. A direction of the gravity components is the vertical direction. Incidentally, the evaluating unit 42 may apply a low-pass filter to the accelerations in the three-axis directions at each measurement time in order to separate the gravity components, and may use components obtained by subtracting the gravity components from the accelerations in the three-axis directions at each measurement time as the oscillating components due to movement of the horse.

Incidentally, gaits of the horse in movement are mainly classified into a walk, a trot, a canter, and a gallop. The speed of the horse increases in order of the walk, the trot, the canter, and the gallop, and oscillation of the horse at the time of running is increased accordingly, so that changes in the accelerations in the three-axis directions are also increased. Furthermore, in the trot, the canter, and the gallop, the body of the horse floats in the air during one stride, and a zero-gravity state with no accelerations in the three-axis directions occurs. Therefore, the evaluating unit 42 separates the gravity components by using data corresponding to a period in which the changes in the accelerations in the three-axis directions are small.

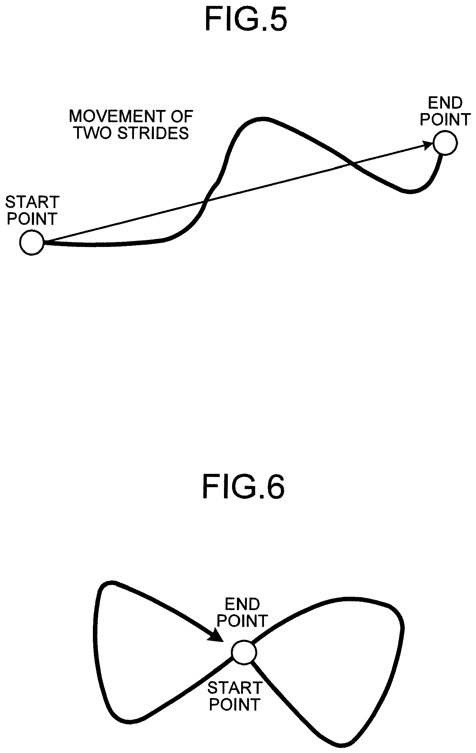

Then, the evaluating unit 42 obtains a trajectory of the position of the brisket of the horse by using the oscillating components due to the movement of the horse in the accelerations in the three-axis directions at each measurement time and by using the angular velocities of the three axes. For example, the evaluating unit 42 calculates a three-dimensional position at each measurement time by using the oscillating components due to the movement of the horse in the three-axis directions at each measurement time and by using the angular velocities of the three axes. Consequently, a trajectory of the three-dimensional position is obtained. FIG. 5 is a diagram illustrating an example of a trajectory of positions. The evaluating unit 42 specifies a plurality of feature points at which the oscillating components measured from respective positions have the same feature. For example, the evaluating unit 42 specifies, as the feature point, a point at which the oscillation is the greatest or a point at which the acceleration in the vertical direction is the greatest. In the example in FIG. 5, a start point and an end point are specified as the feature points. The evaluating unit 42 performs coordinate transformation of positions between the feature points onto a plane such that the feature points are located in the same position, and obtains a trajectory of the positions on the plane. For example, the evaluating unit 42 projects the positions between the feature points onto a vertical plane connecting the feature points, and obtains a trajectory of the position of the brisket of the horse on the plane viewed from the front of the horse. In the example in FIG. 5, the trajectory of the position of the brisket of the horse is obtained by projecting the coordinates of the respective positions between the start point and the end point onto the vertical plane connecting the positions of the start point and the end point. FIG. 6 is a diagram illustrating an example of the trajectory of the position of the brisket of the horse on the plane. In the example in FIG. 6, the start point and the end point of the trajectory are located in the same position.

The method of obtaining the trajectory of the position of the brisket of the horse on the plane is not limited to the above example. For example, the evaluating unit 42 may obtain an up-down direction of the horse, a left-right direction of the horse, and a front-back direction of the horse by using characteristics of walking of the horse, and may obtain the trajectory on the plane by using an oscillating component in the up-down direction of the horse and an oscillating component in the left-right direction of the horse. For example, the evaluating unit 42 separates the oscillating component in the up-down direction from the oscillating components due to the movement of the horse by assuming that a component in the vertical direction is the up-down direction of the horse. Furthermore, if the moving speed of the horse is steady, movement of the horse in the left-right direction is large but movement of the horse in the front-back direction is small. Therefore, the evaluating unit 42 determines, as the left-right direction of the horse, a direction in which oscillation is the greatest among directions perpendicular to the vertical direction, with respect to the oscillating components due to the movement of the horse except for the oscillating component in the up-down direction. Moreover, the evaluating unit 42 determines, as the front-back direction of the horse, a direction perpendicular to both of the up-down direction of the horse and the left-right direction of the horse. The evaluating unit 42 may separate the oscillating component in the left-right direction of the horse and an oscillating component in the front-back direction of the horse from the oscillating components due to the movement of the horse, and obtain the trajectory on the plane viewed from the front of the horse by using the oscillating component in the left-right direction and the oscillating component in the front-back direction. Incidentally, the method of obtaining the left-right direction of the horse and the front-back direction of the horse is not limited to the above example.

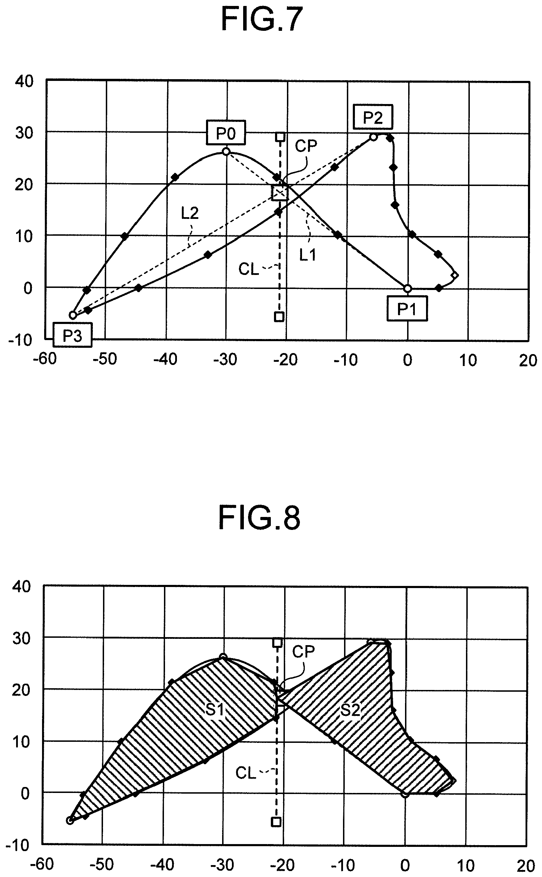

The evaluating unit 42 evaluates the balance between right and left in movement of the horse by using the trajectory of the position of the brisket of the horse on the plane. FIG. 7 is a diagram illustrating an example of the trajectory of the position of the brisket of the horse. In the example in FIG. 7, the horizontal axis represents an amount of movement in the horizontal direction in units of millimeters (mm), and serves as the X-axis. The vertical axis represents an amount of movement in the up-down direction in units of mm, and serves as the Y-axis. In the first embodiment, the balance between right and left is evaluated from the amount of change in a position of a point; therefore, any point can be used as a reference point (point that serves as zero) of the X-axis and the Y-axis. The example in FIG. 7 illustrates a trajectory when the gait of the horse is the trot. It is assumed that the trajectory illustrated in FIG. 7 is measured in sequence of a point P0 → a point P1 → a point P2 → a point P3 in order of the measurement time. As illustrated in FIG. 7, a trajectory of the position of the brisket generally has a shape like a sign of infinity ($\infty$, that is, laterally facing 8-shape). The reason for this may be that the horse keeps the balance by moving the head in the 8-shape while walking. In contrast, if the horse has lameness, the trajectory is deformed from the shape like a sign of infinity because the horse favors a leg with the lameness. Therefore, the evaluating unit 42 evaluates the balance between right and left of the trajectory. A method of evaluating the balance between right and left of the trajectory will be described in detail below.

The evaluating unit 42 obtains the point P0 that is a maximum point on the left side of the trajectory of position of the brisket, the point P1 that is a minimum point on the right side, the point P2 that is a maximum point on the right side, and the point P3 that is a minimum point on the left side. For example, the evaluating unit 42 compares the coordinates of the positions of the respective points in order of the measurement time, and sequentially determines a maximum point, for which the position of a next point in the up-down direction first decreases, and a minimum point, for which the position of a next point in the up-down direction first increases. Then, if a point that is determined as the minimum point next to the maximum point is located on the right side of the maximum point, the evaluating unit 42 determines this maximum point as the maximum point on the left side and determines this minimum point as the minimum point on the right side. Furthermore, if a point that is determined as the minimum point next to the maximum point is located on the left side of the maximum point, the evaluating unit 42 determines this maximum point as the maximum point on the right side and determines the minimum point as the minimum point on the left side. As a method of specifying the maximum points on the left side and the right side, it may be possible to determine that a maximum point is located on the left side when the X coordinate of the maximum point is smaller than the X coordinate of an intersection CP and determine that a maximum point is located on the right side when the X coordinate of the maximum point is greater than the X coordinate of the intersection CP. The trajectory of the point P0 → the point P1 → the point P2 → the point P3 → the point P0 illustrated in FIG. 7 corresponds to a single stride. The evaluating unit 42 repeatedly specifies points in order of the point P0 → the point P1 → the point P2 → the point P3 → the point P0 ..., and obtains a trajectory of each stride. Incidentally, a point serving as a start point of the trajectory of a single stride may be any of the points P0 to P3.

The evaluating unit 42 evaluates the balance between right and left of the trajectory for each stride. For example, the evaluating unit 42 obtains a line segment L1 connecting the point P0 and the point P1 and a line segment L2 connecting the point P2 and the point P3. The evaluating unit 42 obtains the intersection CP the line segment L1 and the line segment L2.

For example, it is assumed that the XY coordinate of the point P0 is (P0.X, P0.Y), the XY coordinate of the point P1 is (P1.X, P1.Y), the XY coordinate of the point P2 is (P2.X, P2.Y), and the XY coordinate of the point P3 is (P3.X, P3.Y). In this case, the XY coordinate of the intersection CP (intersection.X, intersection.Y) is obtained as follows.

$$A = P1.Y - P0.Y$$

$$B = P0.X - P1.X$$

$$U = (P1.Y - P0.Y) \times P0.X - (P1.X - P0.X) \times P0.Y$$

$$C = P3.Y - P2.Y$$

$$D = P2.X - P3.X$$

$$V = (P3.Y - P2.Y) \times P2.X - (P3.X - P2.X) \times P2.Y$$

$$intersection.X = (D \times U - B \times V) / (A \times D - B \times C)$$

$$intersection.Y = (A \times V - C \times U) / (A \times D - B \times C)$$

The evaluating unit 42 obtains a vertical center line CL that passes through the intersection CP and that extends in the up-down direction. Then, the evaluating unit 42 calculates an index 1 indicating an area ratio of a left side and a right side of the trajectory with respect to the center line CL. Furthermore, the evaluating unit 42 calculates an index 2 indicating a length ratio of a left portion and a right portion of the line segment L1 and the line segment L2 with respect to the center line CL. Moreover, the evaluating unit 42 calculates an index 3 indicating a reduction amount ratio of the point P1 and the point P3 with respect to the intersection CP. Incidentally, the indices 1 to 3 are examples, and any index may be used as long as it indicates the balance between right and left of the trajectory. A method of obtaining the indices 1 to 3 will be described in detail below.

FIG. 8 is a diagram illustrating an example of a left area and a right area of the trajectory with respect to the center line CL. In the example in FIG. 8, a left area S1 of the trajectory with respect to the center line CL and a right area S2 of the trajectory with respect to the center line CL are illustrated. For example, assuming that the trajectory includes N points, and the coordinates of the N points are $(x_j, y_j)$, an area S is obtained by Expression (1) below.

$$S = \frac{1}{2} \left| \sum_{j=1}^{n} (x_j - x_{j+1}) \times (y_j + y_{j+1}) \right| \tag{1}$$

The evaluating unit 42 calculates, as the left area S1 of the trajectory, an approximate value of an area of a polygon formed by left points of the trajectory and the intersection CP by substituting the coordinates of the left points of the trajectory and the intersection CP into Expression (1). The evaluating unit 42 calculates, as the right area S2 of the trajectory, an approximate value of an area of a polygon formed by right points of the trajectory and the intersection CP by substituting the coordinates of the right points of the trajectory and the intersection CP into Expression (1). The evaluating unit 42 calculates, as a value of the index 1, a value by dividing the left area S1 of the trajectory by the right area S2 of the trajectory.

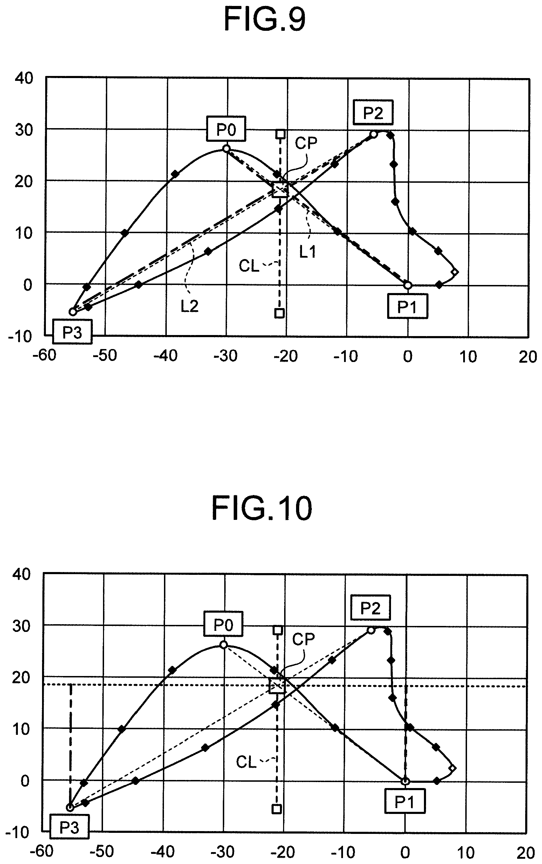

FIG. 9 is a diagram illustrating an example of left portions and right portions of the line segment L1 and the line segment L2 with respect to the center line CL. In the example in FIG. 9, the left portions of the line segment L1 and the line segment L2 with respect to the center line CL are indicated by chain lines. Furthermore, the right portions of the line segment L1 and the line segment L2 with respect to the center line CL are indicated by dashed lines. For example, assuming that the XY coordinate of a point A is (A.X, A.Y) and the XY coordinate of a point B is (B.X, B.Y), a line segment length (A, B) as the length of a line segment between the point A and the point B is represented by Expression (2) below.

$$\text{Line segment length (A, B)} = [(A.X - B.X)^2 + (A.Y - B.Y)^2]^{1/2} \tag{2}$$

The evaluating unit 42 calculates a left line segment length of a left portion of the line segment L1 and the line segment L2 with respect to the center line CL by Expression (3) below. Furthermore, the evaluating unit 42 calculates a right line segment length of a right portion of the line segment L1 and the line segment L2 with respect to the center line CL by Expression (4) below.

$$\text{Left line segment length: line segment length (P0, intersection) + line segment length (P3, intersection)} \tag{3}$$

$$\text{Right line segment length : line segment length (P2, intersection) + line segment length (P1, intersection)} \tag{4}$$

The evaluating unit 42 calculates, as a value of the index 2, a value by dividing the left line segment length by the right line segment length.

FIG. 10 is a diagram illustrating an example of reduction amounts of the point P1 and the point P3 from the intersection CP. In the example in FIG. 10, a portion corresponding to the reduction amount of the point P1 from the intersection CP is indicated by a dashed line. Furthermore, a portion corresponding to the reduction amount of the point P3 from the intersection CP is indicated by a chain line.

The evaluating unit 42 calculates a right reduction amount of the point P1 from the intersection CP by Expression (5) below. The evaluating unit 42 calculates a left reduction amount of the point P3 from the intersection CP by Expression (6) below.

$$\text{Right reduction amount: } |intersection.Y - P1.Y| \quad (5)$$

$$\text{Left reduction amount: } |intersection.Y - P3.Y| \quad (6)$$

The evaluating unit 42 calculates, as a value of the index 3, a value by dividing the left reduction amount by the right reduction amount.

The evaluating unit 42 evaluates the balance between right and left of the trajectory for each trajectory of a single stride by using the indices 1 to 3. For example, the evaluating unit 42 evaluates whether each balance is on the left side or the right side on the basis of the indices 1 to 3. The indices 1 to 3 are ratios of the left side to the right side; therefore, it is possible to evaluate that the balance is off to the right side when the values are smaller than 1 and the balance is off to the left side when the values are greater than 1. The evaluating unit 42 evaluates the balance between right and left in movement, by a majority voting of evaluations on whether the balance is on the left side or the right side based on the indices 1 to 3.

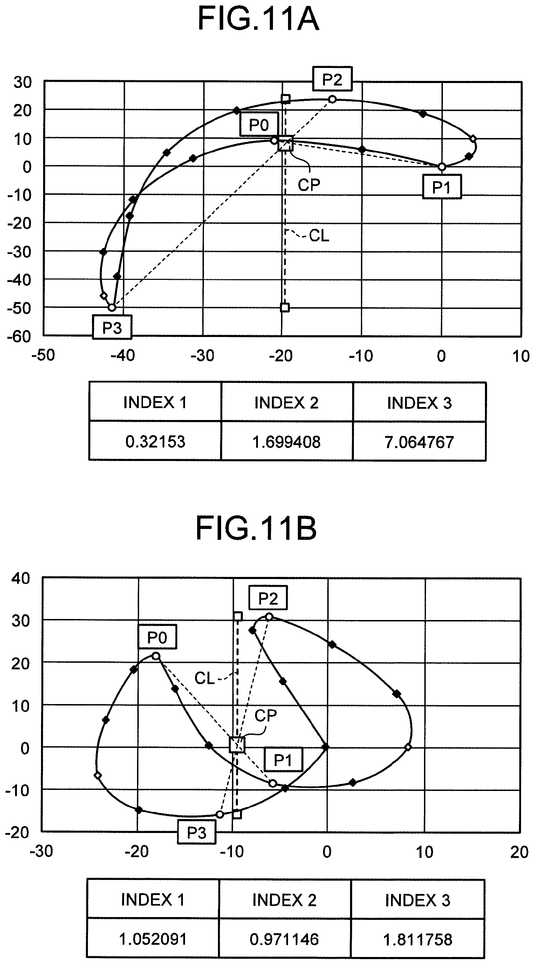

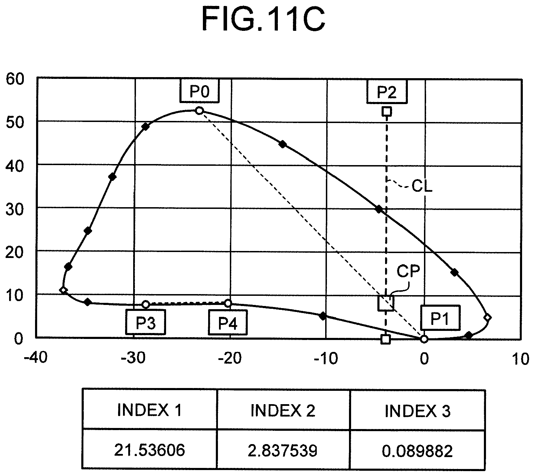

Incidentally, the balance between right and left changes due to a change in the road surface during walking or movement of the horse, and the trajectory also changes accordingly. Therefore, in some cases, it may be difficult to appropriately evaluate the balance between right and left by individually using each of the indices 1 to 3. FIG. 11A to FIG. 11C are diagrams illustrating examples in which the balance between right and left is not appropriately evaluated. In the example in FIG. 11A, it is determined that the balance is off to the right side when only the index 1 is used because its value is smaller than 1. However, in reality, the balance is off to the left side in the example in FIG. 11A, and this can be accurately determined by using the index 2 and the index 3. In the example in FIG. 11B, it is determined that the balance is off to the right side when only the index 2 is used because its value is smaller than 1. However, in reality, the balance is off to the left side in the example in FIG. 11B, and this can be accurately determined by using the index 1 and the index 3. In the example in FIG. 11C, it is determined that the balance is off to the right side when only the index 3 is used because its value is smaller than 1. However, in reality, the balance is off to the left side in the example in FIG. 11C, and this can be accurately determined by using the index 1 and the index 2. As described above, the evaluating unit 42 can evaluate the balance between right and left in movement with accuracy by performing a majority voting of evaluations based on the indices 1 to 3.

The determining unit 43 performs various determinations. For example, the determining unit 43 determines the gait of the horse on the basis of the measurement data 35. For example, the determining unit 43 obtains, from the measurement data 35, a value $\alpha$ of an acceleration in the up-down direction and a square $\beta$ of the absolute value of the acceleration, and determines whether a gait in which the horse moves is the walk, the trot, the canter, or the gallop by using $\alpha$ and $\beta$. Details of the determination of the gait is described in Japanese Laid-open Patent Publication No. 2015-84943 disclosed by the applicant of the present application, and therefore, explanation thereof will be omitted.

The evaluating unit 42 stores, as the evaluation data 36, an evaluation result of the balance between right and left of the trajectory for each stride and the gait determined by the determining unit 43 in the storage unit 33. For example, the evaluating unit 42 stores, as the evaluation result, a result of the majority voting of the indices 1 to 3 for each stride in the evaluation data 36.

The output unit 44 performs various kinds of output. For example, the output unit 44 outputs evaluation results of a plurality of strides stored in the evaluation data 36. For example, the output unit 44 outputs, to the display unit 31, a screen indicating a distribution ratio of the balance between right and left for a plurality of strides. Furthermore, the output unit 44 outputs, to the display unit 31, a screen indicating a transition of the evaluation result of the balance between right and left for each stride.

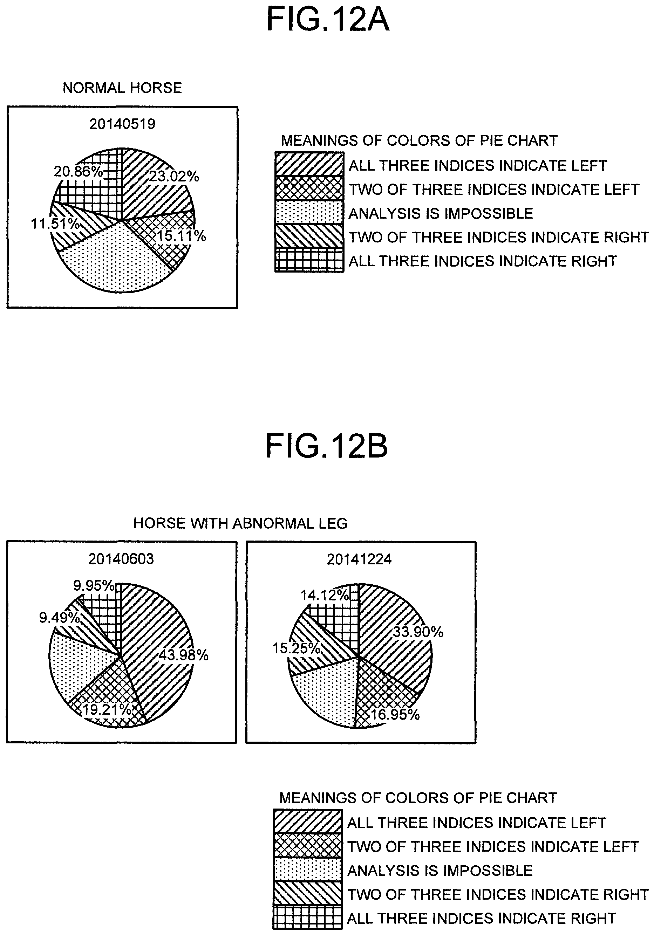

FIG. 12A is a diagram illustrating an example of a distribution ratio of the balance between right and left. In the example in FIG. 12A, a distribution ratio of the balance between right and left for a plurality of strides of a normal horse without lameness is illustrated by a pie chart. The balance between right and left changes due to a change in the road surface during walking or movement of the horse. Therefore, the balance between right and left changes for each stride, but the balance between right and left of a normal horse is approximately equally distributed when a plurality of strides are observed. FIG. 12B is a diagram illustrating examples of a distribution ratio of the balance between right and left. In the examples in FIG. 12B, a distribution ratio of the balance between right and left for a plurality of strides of a horse with lameness is illustrated by a pie chart. FIG. 12B illustrates two examples. If an abnormality occurs in a leg, a horse walks while favoring the leg so as not to bear the weight on the leg in which the abnormality has occurred. Therefore, when a plurality of strides are observed, the balance between right and left is lost, and a percentage at which it is determined that the balance is off to a non-favored leg side (a side where the abnormality has not occurred) is increased. In the example in FIG. 12B, a percentage at which it is determined that the balance is off to the left side is increased, so that is is estimated that an abnormality has occurred in the right leg when viewed from the front of the horse.

Furthermore, it may be possible to recognize a daily change in the condition by arranging, in chronological order, distribution ratios of the balance between right and left that are aggregated for a plurality of strides for each day, and comparing the distribution ratios. For example, as illustrated in FIG. 12B, distribution ratios at different timings are arranged and displayed side by side. It is assumed that the distribution ratio on the left side of FIG. 12B is obtained on June 3, and the distribution ratio on the right side of FIG. 12 is obtained on December 24. In the example in FIG. 12B, it is indicated that a state largely inclined to the left side on June 3 is recovered on December 24. For example, it may be possible to cause the accepting unit 41 to display a specification screen for specifying dates for comparing evaluations and accept specified dates for comparison, and cause the output unit 44 to display distribution ratios of the specified dates side by side.

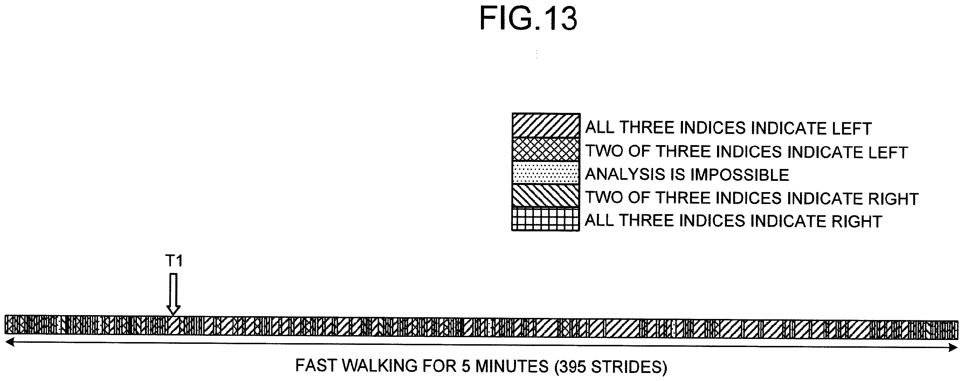

FIG. 13 is a diagram illustrating an example of a transition of an evaluation result of the balance between right and left for each of a plurality of strides. In the example in FIG. 13, evaluation results of the balance between right and left for respective 395 strides during a training of fast walking (trot) for five minutes are sequentially illustrated in order of the measurement time. In the example in FIG. 13, a pattern is changed in accordance with the evaluation result of each stride. In the example in FIG. 13, in the beginning of the training, evaluations indicating that the balance is off to the left side and evaluations indicating that the balance is off to the right side are mixed, and an abnormality is not observed. However, in the example in FIG. 13, evaluations indicating that the balance is off to the left side is increased from a time T1. Therefore, it is estimated that an abnormality has occurred in the right leg when viewed from the front of the horse and lameness has occurred after the time T1.

An administrator who manages the horse can recognize that an abnormality has occurred in the horse by viewing the evaluation results displayed on the display unit 31. Furthermore, the administrator who manages the horse can recognize whether the impact of a training menu on the horse is the same as expected or the impact is too light or too heavy by viewing the evaluation results displayed on the display unit 31. If it is assumed that FIG. 13 illustrates rehabilitative data for a horse with lameness, T1 at which the horse started to incline has occurred when 20% of time has elapsed since a start of the training even though T1 is expected to occur when, for example, 80% of time has elapsed since the start of the training. Therefore, it is found that the training menu is too heavy.

Incidentally, the output unit 44 may output evaluation results for a predetermined gait among the evaluation results stored in the evaluation data 36. The predetermined gait may be set in advance. Furthermore, the accepting unit 41 may display a selection screen for selecting a gait for displaying evaluation results and accept a selection of a gait, and the output unit 44 may output evaluation results for the selected gait. Lameness is likely to be detected in the walk or the trot. Therefore, the output unit 44 may output evaluation results for a gate such as the walk or the trot. Consequently, the evaluation device 12 can detect an occurrence of lameness from the output evaluation results with accuracy.

Flow of Processes

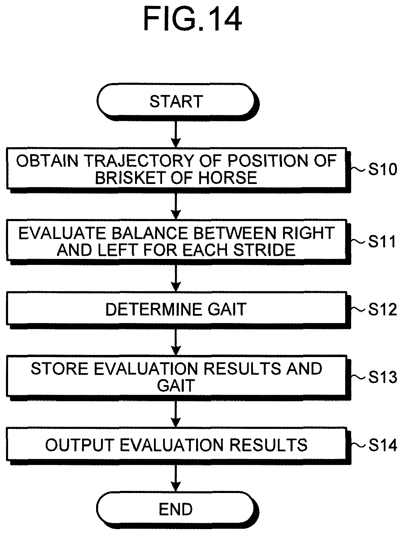

The flow of an evaluation process of evaluating the balance between right and left in movement for each stride from the measurement data 35 by the evaluation device 12 according to the first embodiment will be described below. FIG. 14 is a flowchart illustrating an example of the flow of the evaluation process. The evaluation process is performed at a predetermined timing, such as a timing at which an instruction to start evaluation is accepted from the input unit 32.

As illustrated in FIG. 14, the evaluating unit 42 reads the measurement data 35, and obtains a trajectory of the position of the brisket of a horse on a plane from the measurement data 35 (S10). The evaluating unit 42 sequentially obtains, from the trajectory, the point P0 as the maximum point on the left side of the trajectory of the position of the brisket, the point P1 as the minimum point on the right side, the point P2 as the maximum point on the right side, and the point P3 as the minimum point on the left side, and evaluates the balance between right and left of the trajectory for each stride (S11).

The determining unit 43 obtains, from the measurement data 35, the value $\alpha$ of an acceleration in the up-down direction and the square $\beta$ of the absolute value of the acceleration, and determines a gait in which the horse moves by using $\alpha$ and $\beta$ (S12).

The evaluating unit 42 stores, as the evaluation data 36, an evaluation result of the balance between right and left of the trajectory for each stride and the gait determined by the determining unit 43 in the storage unit 33 (S13).

The output unit 44 outputs evaluation results of a plurality of strides stored in the evaluation data 36 (S14), and the process ends.

Incidentally, in the evaluation process, it may be possible to first determine a gait and then analyze a trajectory for data of the gait.

Effects

As described above, the evaluation device 12 according to the first embodiment evaluates the balance between right and left in movement, for each stride on the basis of the measurement data 35 of the sensor unit 21 worn by a horse that moves by four legs. The evaluation device 12 outputs evaluation results for a plurality of strides. Therefore, the evaluation device 12 can easily detect lameness.

Furthermore, the evaluation device 12 according to the first embodiment obtains a trajectory of the position of the brisket of an animal for each stride, on the basis of the measurement data 35. The evaluation device 12 calculates an area ratio of a left side and a right side of the trajectory with respect to the vertical center line CL that passes through the intersection CP of the line segment L1 connecting the maximum point on the left side of the trajectory and the minimum point on the right side and the line segment L2 connecting the maximum point on the right side of the trajectory and the minimum point on the left side. Furthermore, the evaluation device 12 calculates a length ratio of a left portion and a right portion of the line segment L1 and the line segment L2 with respect to the center line CL. The evaluation device 12 calculates a reduction amount ratio of the minimum point on the right side and the minimum point on the left side with respect to the intersection CL. The evaluation device 12 evaluates the balance between right and left in movement by using the area ratio, the length ratio, and the reduction amount ratio. Therefore, the evaluation device 12 can appropriately evaluate the balance between right and left even for a trajectory in which it is difficult to determine the balance between right and left.

Furthermore, the evaluation device 12 according to the first embodiment evaluates whether the balance is off to the left side or the right side for each stride on the basis of the area ratio, the length ratio, and the reduction amount ratio, and evaluates the balance between right and left in movement by a majority voting of evaluations. Therefore, the evaluation device 12 can evaluate the balance between right and left in movement with accuracy.

Moreover, the evaluation device 12 according to the first embodiment outputs one of a distribution ratio of the balance between right and left for a plurality of strides and transitions of evaluation results of the balance between right and left for a plurality of strides. Therefore, the evaluation device 12 can output data such that an occurrence of lameness can easily be recognized.

Furthermore, the evaluation device 12 according to the embodiment determines a gait of a horse on the basis of the measurement data 35. The evaluation device 12 according to the first embodiment outputs an evaluation result for a predetermined gait. Therefore, the evaluation device 12 can detect an occurrence of lameness with accuracy.

[b] Second Embodiment

Next, a second embodiment will be described. In the second embodiment, a case will be described in which the evaluation device 12 is a mobile terminal. A configuration of the system 10 according to the second embodiment is the same as the first embodiment illustrated in FIG. 1 and FIG. 3, and therefore, explanation thereof will be omitted.

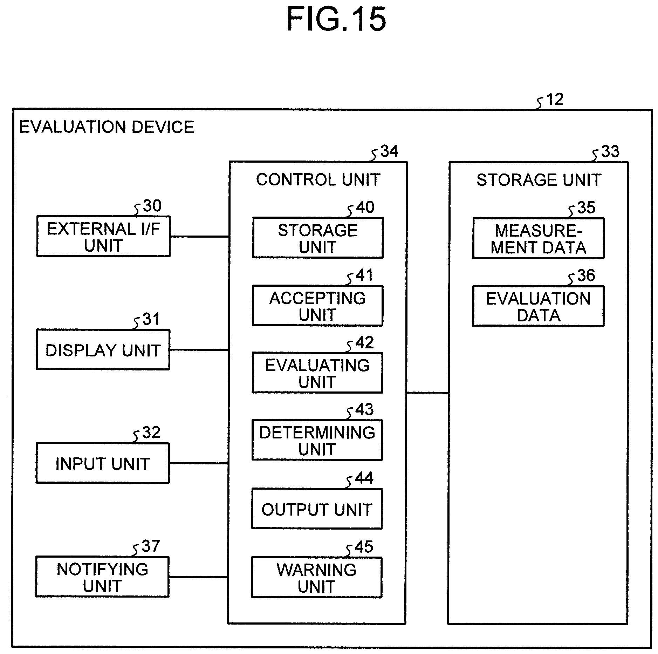

FIG. 15 is a diagram illustrating an example of a functional configuration of an evaluation device according to the second embodiment. A configuration of the evaluation device 12 according to the second embodiment is approximately the same as the first embodiment illustrated in FIG. 4; therefore, the same components will be denoted by the same signs and a difference will mainly be described below.

As illustrated in FIG. 15, the evaluation device 12 further includes a notifying unit 37.

The notifying unit 37 is a device that gives a notice. For example, the notifying unit 37 may be a vibrator that gives a notice by vibration or a speaker that gives a notice by sound.

The control unit 34 further includes a warning unit 45.

The warning unit 45 gives various warnings. For example, if a state in which the balance between right and left is off to either side is detected in evaluations of a plurality of strides performed by the evaluating unit 42, the warning unit 45 controls the notifying unit 37 and gives a warning. For example, if a ratio of the left side or the right side in evaluations for the latest predetermined period or for a predetermined number of strides is equal to or greater than a predetermined threshold (for example, 80%) that is used to determine that the balance is off to either side, the warning unit 45 gives a warning. The threshold may be set from outside. Incidentally, the warning unit 45 may give a warning when evaluations of one of the left side and the right side by the evaluating unit 42 are continued a predetermined number of times, such as five times.

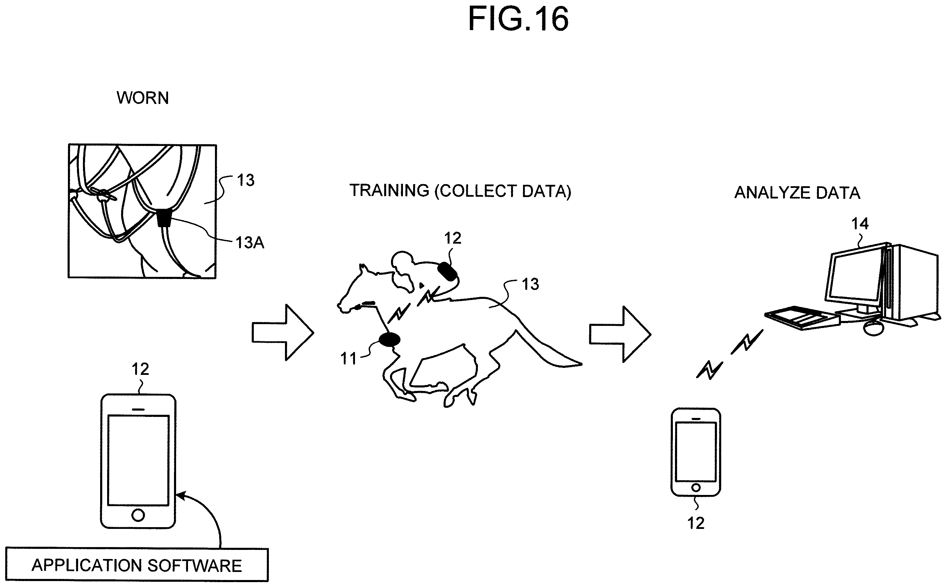

FIG. 16 is a diagram illustrating an example of the flow of managing the health of a horse by a system according to the second embodiment. The measuring device 11 is put on the brisket of the horse 13 to be subjected to health management. Furthermore, for example, an application software is installed in a smartphone to cause the smartphone to function as the evaluation device 12.

A person responsible for training of the horse 13 carries the evaluation device 12 and trains the horse 13. The evaluation device 12 and the measuring device 11 are enabled to communicate with each other through short-distance wireless communication, such as Bluetooth (registered trademark). The evaluation device 12 continually receives measurement data 24 from the measuring device 11 and evaluates the balance between right and left in real time. If a state in which the balance between right and left is off to either side is detected in evaluations of a plurality of strides as a result of evaluations, the evaluation device 12 gives a warning. Therefore, the evaluation device 12 can detect lameness and gives a warning in real time during a training, so that it is possible to detect an occurrence of an abnormality at an early stage. For example, if lameness is detected during a training as illustrated in FIG. 13, the evaluation device 12 can stop the training by giving a warning, so that it is possible to prevent an abnormality in the leg from getting worse.

After the training, the evaluation device 12 is brought to an office of the management source, and the measurement data 35 and the evaluation data 36 are uploaded on a terminal device 14 via a storage medium or through wire communication or wireless communication. The terminal device 14 manages the uploaded measurement data 35 and the uploaded evaluation data 36. Incidentally, the terminal device 14 may perform a detailed analysis of the health management of the horse 13 by using the measurement data 35 and the evaluation data 36.

Effects

As described above, if a state in which the balance between right and left is off to either side is detected in evaluations of a plurality of strides, the evaluation device 12 according to the second embodiment gives a warning. Therefore, when an abnormality has occurred in a leg, the evaluation device 12 can prevent the abnormality from getting worse.

[c] Third Embodiment

While the embodiments of the devices of the disclosed technology have been explained above, the disclosed technology may be embodied in various forms other than the embodiments as described above. Therefore, the other embodiments of the present invention will be explained below.

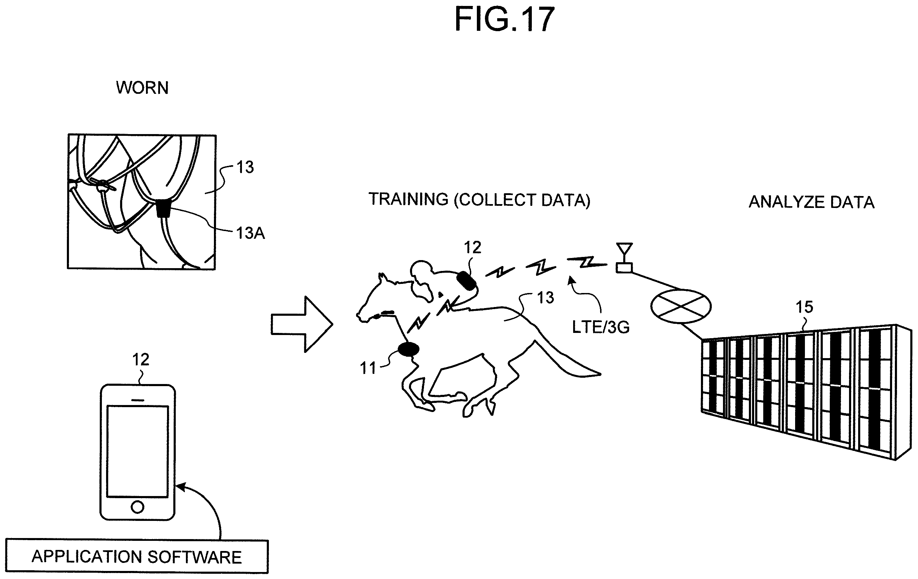

For example, in the above-described second embodiment, an example has been described in which the evaluation device 12 uploads the measurement data 35 and the evaluation data 36 on the terminal device 14 installed in the office of the management source; however, it is not limited thereto. For example, the evaluation device 12 may upload the measurement data 35 and the evaluation data 36 on a server device in the cloud. FIG. 17 is a diagram illustrating an example of the flow of managing the health of a horse by a system according to a third embodiment. For example, the evaluation device 12 uploads the measurement data 35 and the evaluation data 36 on a server device 15 in the cloud through a mobile communication network or the like. The server device 15 manages the uploaded measurement data 35 and the uploaded evaluation data 36. Furthermore, the server device 15 performs a detailed analysis of the health management of the horse 13 by using the measurement data 35 and the evaluation data 36. As described above, by causing the server device 15 in the cloud to manage and analyze the measurement data 35 and the evaluation data 36, a trainer or the like can recognize a health condition of the horse 13 by accessing the server device 15 even when the trainer is outing.

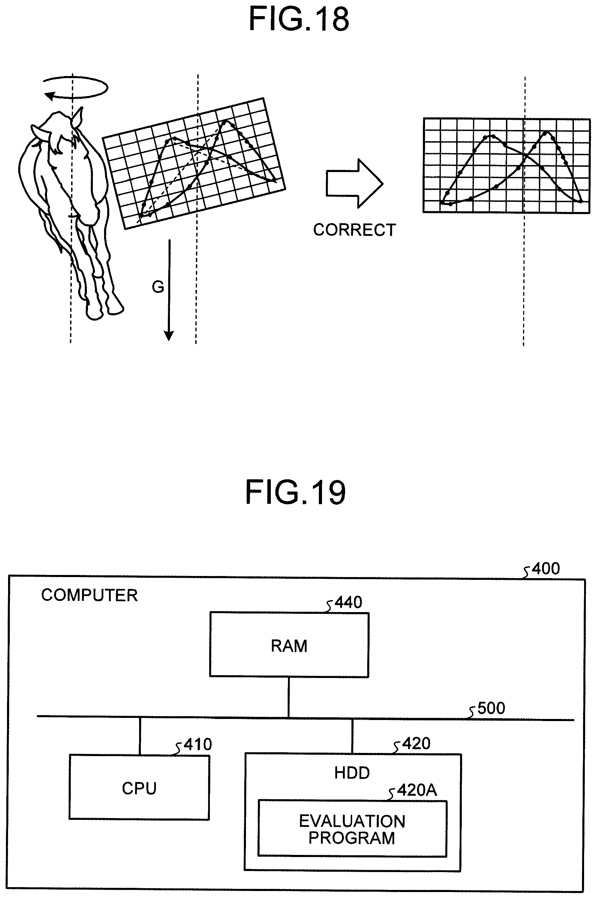

Incidentally, when a horse moves circularly at the time of cornering in a track course or performing a circular movement in a longe, the horse inclines its body to the center of the circle so as not to be influenced by an acceleration in the lateral direction, such as a centrifugal force. Therefore, the balance between right and left of the horse tends to be inclined to the inside of the circular movement and the entire trajectory tends to be inclined. FIG. 18 is a diagram schematically illustrating inclination of a trajectory of a circular movement. In the circular movement as described above, an angular velocity deviated to one side is detected. Therefore, if the angular velocity deviated to one side is detected, the evaluating unit 42 corrects the trajectory in accordance with the angular velocity. For example, if an angular velocity deviated with respect to an axis in the up-down direction of the horse is detected for a predetermined time longer than a single stride of the horse, the evaluating unit 42 corrects the trajectory so as to rotate the entire trajectory by increasing an angle of rotation with an increase in the angular velocity. If an angular velocity in the clockwise direction is detected with respect to the axis in the up-down direction of the horse, the evaluating unit 42 rotates the entire trajectory to the right. If an angular velocity in the counterclockwise direction is detected with respect to the axis in the up-down direction of the horse, the evaluating unit 42 rotates the entire trajectory to the left. The evaluating unit 42 evaluates the balance between right and left by using the corrected trajectory. Consequently, even when the horse moves circularly, it is possible to correctly evaluate the balance between right and left.

Furthermore, in the above-described embodiments, an example has been described in which the technology is applied to detection of lameness of a horse; however, it is not limited thereto. If any abnormality occurs in a leg of an animal that moves with four legs, the animal favors the leg in which the abnormality has occurred, which leads to lameness. Therefore, the evaluation device 12 may be used to detect lameness of an animal that moves with four legs.

Moreover, in the above-described embodiments, an example has been described in which the balance between right and left in movement of the horse is evaluated from the measurement data measured by the measuring device 11 put on the brisket of the horse; however, it is not limited thereto. The measuring device 11 may be put on anywhere as long as it is possible to evaluate the balance between right and left. That is, because a position appropriate for evaluation of the balance between right and left in movement is different depending on an animal that moves with four legs, the measuring device 11 may be put on a position appropriate for each measurement of a trajectory.

Furthermore, in the above-described embodiments, an example has been described in which the balance between right and left is evaluated by a majority voting of evaluation results of the indices 1 to 3; however, it is not limited thereto, and appropriate modification is possible. For example, the evaluating unit 42 may evaluate the balance between right and left by using any one or two of the indices 1 to 3. In addition, the evaluating unit 42 may evaluate the balance between right and left from a result of weighting of evaluation results of a plurality of indices. For example, the evaluating unit 42 may add a greater weight to an evaluation result of an index for which the evaluation accuracy is higher, in accordance with a moving condition, such as a gait or a load surface, and may evaluate the balance between right and left from a weighted result.

Moreover, the components illustrated in the drawings are functionally conceptual and need not necessarily be physically configured in the manner illustrated in the drawings. That is, specific forms of distribution and integration of the devices are not limited to those illustrated in the drawings, and all or part of the devices may be functionally or physically distributed or integrated in arbitrary units depending on various loads or use conditions. For example, the processing units such as the storage unit 40, the accepting unit 41, the evaluating unit 42, the determining unit 43, the output unit 44, and the warning unit 45 may be integrated appropriately. Furthermore, processes performed by the processing units may be divided into processes of an appropriate number of processing units. Moreover, for each processing function performed by each processing unit, all or any part of the processing function may be implemented by a CPU and a program analyzed and executed by the CPU or may be implemented as hardware by wired logic.

Evaluation Program

Various processes explained in the above-described embodiments may be implemented by causing a computer system, such as a personal computer or a workstation, to execute a program prepared in advance. Therefore, an example of the computer system that executes a program having the same functions as those of the above-described embodiments will be described below.. FIG. 19 is a diagram illustrating an example of a configuration of a computer that executes an evaluation program.

As illustrated in FIG. 19, a computer 400 includes a CPU 410, a hard disk drive (HDD) 420, and a RAM 440. The components 400 to 440 are connected to one another via a bus 500.

In the HDD 420, an evaluation program 420A is stored in advance, which implements the same functions as those of the storage unit 40, the accepting unit 41, the evaluating unit 42, the determining unit 43, the output unit 44, and the warning unit 45 as described above. The evaluation program 420A may be separated appropriately.

Furthermore, the HDD 420 stores therein various kinds of information. For example, the HDD 420 stores therein an OS or various kinds of data.

The CPU 410 reads the evaluation program 420A from the HDD 420, executes the program, and performs the same operations as those of each processing unit of the embodiments. That is, the evaluation program 420A implements the same operations as those of the storage unit 40, the accepting unit 41, the evaluating unit 42, the determining unit 43, the output unit 44, and the warning unit 45.

The above-described evaluation program 420A need not be stored in the HDD 420 from the beginning.

For example, the program may be stored in a "portable physical medium", such as a flexible disk (FD), a compact disk read only memory (CD-ROM), a digital versatile disk (DVD), a magneto-optical disk, or an integrated circuit (IC) card, that is inserted in the computer 400. Then, the computer 400 may read the program from the medium and execute the program.

Furthermore, the program may be stored in "other computers (or servers)" that are connected to the computer 400 via a public line, the Internet, a LAN, or a WAN. Then, the computer 400 may read the program from the computers and execute the program.

According to an embodiment of the present invention, it is possible to easily detect lameness.

**Claims**

1.  An evaluation device comprising:

    an evaluating unit (42) adapted to evaluate balance between right and left in movement, for each stride on the basis of a measurement data obtained by a sensor worn by an animal that moves with four legs; and
    an output unit (44) adapted to output evaluation results of a plurality of strides obtained by the evaluating unit
    wherein the evaluating unit is adapted to obtain a trajectory of a position of a brisket of the animal for each stride on the basis of the measurement data by projecting coordinates of respective positions between a start point and an end point onto a vertical plane connecting the positions of the start point and the end point, **characterised in that** the evaluating unit is further adapted to calculate one of

an area ratio of a left side and a right side of the trajectory with respect to a vertical center line that passes through an intersection of a first line segment, which connects a maximum point on the left side of the trajectory and a minimum point on the right side of the trajectory, and a second line segment, which connects a maximum point on the right side of the trajectory and a minimum point on the left side of the trajectory, the maximum point being a point for which a position of a next point in an up-down direction first decreases and the minimum point being a point for which a position of a next point in the up-down direction first increases, a length ratio of a left portion and a right portion of the first line segment and the second line segment with respect to the center line, the left portion being a sum of a length from the maximum point on the left side to the intersection and a length from the minimum point on the left side to the intersection, and the right portion being a sum of a length from the maximum point on the right side to the intersection and a length from the minimum point on the right side to the intersection, and

a reduction amount ratio which is a ratio between a right reduction amount that is a difference between the minimum point on the right side and the intersection in the up-down direction and a left reduction amount that is a difference between the minimum point on the left side and the intersection in the up-down direction, and evaluate the balance between right and left in movement by using at least one of the area ratio, the length ratio, and the reduction amount ratio.

2. The evaluation device according to claim 1, wherein the evaluating unit is adapted to calculate the area ratio, the length ratio, and the reduction amount ratio for each stride, and evaluating the balance between right and left in movement by using all of the ratios.

3. The evaluation device according to claim 2, wherein the evaluating unit is adapted to evaluate whether the balance is off to the left side or the right side for each stride with respect to each of the area ratio, the length ratio, and the reduction amount ratio, and to evaluate the balance between right and left by majority of an evaluation result of the balance of the left side and the right side.

4. The evaluation device according to any one of claims 1 to 3, wherein the output unit is adapted to output one of a distribution ratio of the balance between right and left for a plurality of strides and transitions of evaluation results of the balance between right and left for a plurality of strides.

5. The evaluation device according to any one of claims 1 to 4, further comprising a warning unit (45) adapted to give a warning when a state in which the balance between right and left is off to one side in evaluations of a plurality of strides performed by the evaluating unit.

6. The evaluation device according to any one of claims 1 to 4, further comprising:

   a determining unit (43) adapted to determine a gait of the animal on the basis of the measurement data, wherein the output unit is adapted to output an evaluation result for a predetermined gait.

7. An evaluation program that causes a computer to execute a process comprising:

   evaluating balance between right and left in movement, for each stride on the basis of measurement data obtained by a sensor worn by an animal that moves with four legs; and
   outputting evaluation results for a plurality of strides wherein the evaluating includes
   obtaining a trajectory of a position of a brisket of the animal for each stride on the basis of the measurement data by projecting coordinates of respective positions between a start point and an end point onto a vertical plane connecting the positions of the start point and the end point, **characterised in**: calculating one of

   an area ratio of a left side and a right side of the trajectory with respect to a vertical center line that passes through an intersection of a first line segment, which connects a maximum point on the left side of the trajectory and a minimum point on the right side of the trajectory, and a second line segment, which connects a maximum point on the right side of the trajectory and a minimum point on the left side of the trajectory, the maximum point being a point for which a position of a next point in an up-down direction first decreases and the minimum point being a point for which a position of a next point in the up-down direction first increases, a length ratio of a left portion and a right portion of the first line segment and the second line segment with respect to the center line, the left portion being a sum of a length from the maximum point on the left side to the intersection and a length from the minimum point on the left side to the intersection, and the right portion being a sum of a length from the maximum point on the right side to the intersection and a length from the minimum point on the right side to the intersection, and

a reduction amount ratio which is a ratio between a right reduction amount that is a difference between the minimum point on the right side and the intersection in the up-down direction and a left reduction amount that is a difference between the minimum point on the left side and the intersection in the up-down direction, and

evaluating the balance between right and left in movement by using at least one of the area ratio, the length ratio, and the reduction amount ratio.

**Patentansprüche**

1. Auswertungsvorrichtung, umfassend:

   eine auswertende Einheit (42), angepasst, um das Gleichgewicht zwischen rechts und links in Bewegung für jeden Schritt auszuwerten, auf der Grundlage von Messdaten, die von einem Sensor ermittelt werden, der von einem Tier getragen wird, das sich auf vier Beinen bewegt, und
   eine Ausgabeeinheit (44), angepasst, um die Auswertungsergebnisse von mehreren Schritten auszugeben, die von der auswertenden Einheit ermittelt wurden,
   wobei die auswertende Einheit angepasst ist, um eine Bewegungsbahn einer Position einer Brust des Tiers für jeden Schritt auf der Grundlage der Messdaten zu ermitteln, durch Projizieren von Koordinaten der jeweiligen Positionen zwischen einen Anfangspunkt und einem Endpunkt auf eine vertikale Ebene und Verbinden der Positionen des Anfangspunkts und des Endpunkts,
   **dadurch gekennzeichnet, dass** die auswertende Einheit des Weiteren angepasst ist, um eines zu berechnen von einem Verhältnis einer linken Seite und einer rechten Seite der Bewegungsbahn in Bezug auf eine vertikale Mittellinie, die durch einen Schnittpunkt eines ersten Liniensegments verläuft, das einen Maximalpunkt an der linken Seite der Bewegungsbahn und einen Minimalpunkt an der rechten Seite der Bewegungsbahn verbindet, und eines zweiten Liniensegments, das einen Maximalpunkt an der rechten Seite der Bewegungsbahn und einen Minimalpunkt an der linken Seite der Bewegungsbahn verbindet, wobei der Maximalpunkt ein Punkt ist, für den sich eine Position eines nächsten Punktes in einer Richtung von oben nach unten zuerst verringert, und der Minimalpunkt ein Punkt ist, für den sich eine Position eines nächsten Punktes in einer Richtung von oben nach unten zuerst erhöht,
   einem Längenverhältnis eines linken Abschnitts und eines rechten Abschnitts des ersten Liniensegments und des zweiten Liniensegments in Bezug auf die Mittellinie, wobei der linke Abschnitt eine Summe einer Länge vom Maximalpunkt an der linken Seite zum Schnittpunkt und einer Länge vom Minimalpunkt an der linken Seite zum Schnittpunkt ist, und der rechte Abschnitt eine Summe einer Länge vom Maximalpunkt an der rechten Seite zum Schnittpunkt und einer Länge vom Minimalpunkt an der rechten Seite zum Schnittpunkt ist, und
   einem Verhältnis des Ausmaßes der Verringerung, das ein Verhältnis zwischen einem rechten Ausmaß der Verringerung ist, das eine Differenz zwischen dem Minimalpunkt an der rechten Seite und dem Schnittpunkt in Richtung von oben nach unten ist, und einem linken Ausmaß der Verringerung, das eine Differenz zwischen dem Minimalpunkt an der linken Seite und dem Schnittpunkt in Richtung von oben nach unten ist, und
   das Auswerten des Gleichgewichts zwischen rechts und links in Bewegung durch Verwenden von mindestens einem von dem Flächenverhältnis, dem Längenverhältnis und dem Verhältnis des Ausmaßes der Verringerung.

2. Auswertungsvorrichtung nach Anspruch 1, wobei die auswertende Einheit angepasst ist, um das Flächenverhältnis, das Längenverhältnis und das Verhältnis des Ausmaßes der Verringerung für jeden Schritt zu berechnen und unter Verwenden aller Verhältnisse das Gleichgewicht zwischen rechts und links in Bewegung auszuwerten.

3. Auswertungsvorrichtung nach Anspruch 2, wobei die auswertende Einheit angepasst ist, um auszuwerten ob das Gleichgewicht für jeden Schritt in Bezug auf das Flächenverhältnis, das Längenverhältnis und das Verhältnis des Ausmaßes der Verringerung zur linken Seite oder zur rechten Seite verlagert ist, und um das Gleichgewicht zwischen rechts und links anhand der Mehrheit eines Auswertungsergebnisses des Gleichgewichts der linken Seite und der rechten Seite auszuwerten.

4. Auswertungsvorrichtung nach einem der Ansprüche 1 bis 3, wobei die Ausgabeeinheit angepasst ist, um eines des Verteilungsverhältnisses des Gleichgewichts zwischen rechts und links für mehrere Schritte und Übergänge von Auswertungsergebnissen des Gleichgewichts zwischen rechts und links für mehrere Schritte auszugeben.

**5.** Auswertungsvorrichtung nach einem der Ansprüche 1 bis 4, des Weiteren umfassend eine Warneinheit (45), die angepasst ist, um eine Warnung abzugeben bei einem Zustand, in dem das Gleichgewicht zwischen rechts und links bei Auswertungen von mehreren Schritten, die von der auswertenden Einheit vorgenommen wurden, auf eine Seite verlagert ist.

**6.** Auswertungsvorrichtung nach einem der Ansprüche 1 bis 4, des Weiteren umfassend:
eine Bestimmungseinheit (43), die angepasst ist, um eine Gangart des Tieres auf der Grundlage der Messdaten zu bestimmen, wobei die Ausgabeeinheit angepasst ist, um ein Auswertungsergebnis für eine bestimmte Gangart auszugeben.

**7.** Auswertungsprogramm, das verursacht, dass ein Computer einen Prozess ausführt, umfassend:

das Auswerten des Gleichgewichts zwischen rechts und links in Bewegung für jeden Schritt, auf der Grundlage von Messdaten, die von einem Sensor ermittelt werden, der von einem Tier getragen wird, das sich auf vier Beinen bewegt, und
das Ausgeben der Auswertungsergebnisse für mehrere Schritte, wobei das Auswerten das Ermitteln einer Bewegungsbahn einer Position der Brust des Tiers für jeden Schritt auf der Grundlage der Messdaten durch Projizieren von Koordinaten der jeweiligen Positionen zwischen einen Anfangspunkt und einem Endpunkt auf eine vertikale Ebene und Verbinden der Positionen des Anfangspunkts und des Endpunkts umfasst,
**gekennzeichnet durch**:

das Berechnen von einem
eines Flächenverhältnisses an einer linken Seite und einer rechten Seite der Bewegungsbahn in Bezug auf eine vertikale Mittellinie, die durch einen Schnittpunkt eines ersten Liniensegments verläuft, das einen Maximalpunkt an der linken Seite der Bewegungsbahn und einen Minimalpunkt an der rechten Seite der Bewegungsbahn verbindet, und eines zweiten Liniensegments, das einen Maximalpunkt an der rechten Seite der Bewegungsbahn und einen Minimalpunkt an der linken Seite der Bewegungsbahn verbindet, wobei der Maximalpunkt ein Punkt ist, für den sich eine Position eines nächsten Punktes in einer Richtung von oben nach unten zuerst verringert, und der Minimalpunkt ein Punkt ist, für den sich eine Position eines nächsten Punktes in einer Richtung von oben nach unten zuerst erhöht, eines Längenverhältnisses eines linken Abschnitts und eines rechten Abschnitts des ersten Liniensegments und des zweiten Liniensegments in Bezug auf die Mittellinie, wobei der linke Abschnitt eine Summe einer Länge vom Maximalpunkt an der linken Seite zum Schnittpunkt und einer Länge vom Minimalpunkt an der linken Seite zum Schnittpunkt ist, und der rechte Abschnitt eine Summe einer Länge vom Maximalpunkt an der rechten Seite zum Schnittpunkt und einer Länge vom Minimalpunkt an der rechten Seite zum Schnittpunkt ist, und eines Verhältnisses des Ausmaßes der Verringerung, das ein Verhältnis zwischen einem rechten Ausmaß der Verringerung ist, das eine Differenz zwischen dem Minimalpunkt an der rechten Seite und dem Schnittpunkt in Richtung von oben nach unten ist, und einem linken Ausmaß der Verringerung, das eine Differenz zwischen dem Minimalpunkt an der linken Seite und dem Schnittpunkt in Richtung von oben nach unten ist, und
das Auswerten des Gleichgewichts zwischen rechts und links in Bewegung durch Verwenden von mindestens einem von dem Flächenverhältnis, dem Längenverhältnis und dem Verhältnis des Ausmaßes der Verringerung.

## Revendications

**1.** Dispositif d'évaluation comprenant :

une unité d'évaluation (42) adaptée pour évaluer un équilibre entre la droite et la gauche pendant un déplacement, pour chaque pas sur la base de données de mesure obtenues par un capteur porté par un animal qui se déplace à quatre pattes ; et
une unité de sortie (44) adaptée pour délivrer en sortie des résultats d'évaluation d'une pluralité de pas obtenus par l'unité d'évaluation,
dans lequel l'unité d'évaluation est adaptée pour
obtenir une trajectoire d'une position d'un bréchet de l'animal pour chaque pas sur la base des données de mesure en projetant des coordonnées de positions respectives entre un point de départ et un point d'arrivée sur un plan vertical reliant les positions du point de départ et du point d'arrivée,
**caractérisé en ce que** l'unité d'évaluation est en outre adaptée pour
calculer l'un parmi

un rapport de surface d'un côté gauche et d'un côté droit de la trajectoire par rapport à une ligne centrale verticale qui passe à travers une intersection d'un premier segment linéaire, qui relie un point maximal sur le côté gauche de la trajectoire et un point minimal sur le côté droit de la trajectoire, et un second segment linéaire, qui relie un point maximal sur le côté droit de la trajectoire et un point minimal sur le côté gauche la trajectoire, le point maximal étant un point pour lequel une position d'un point suivant dans une direction haut-bas commence par diminuer, et le point minimal étant un point pour lequel une position d'un point suivant dans la direction haut-bas commence par augmenter,

un rapport de longueur d'une portion gauche et d'une portion droite du premier segment linéaire et du second segment linéaire par rapport à la ligne centrale, la portion gauche étant une somme d'une longueur à partir d'un point maximal sur le côté gauche jusqu'à l'intersection et d'une longueur à partir d'un point minimal sur le côté gauche jusqu'à l'intersection, et la portion droite étant une somme d'une longueur à partir d'un point maximal sur le côté droit jusqu'à l'intersection et d'une longueur à partir d'un point minimal sur le côté droit jusqu'à l'intersection, et

un rapport de quantité de réduction qui est un rapport entre une quantité de réduction droite qui est une différence entre le point minimal sur le côté droit et l'intersection dans la direction haut-bas et une quantité de réduction gauche qui est une différence entre le point minimal sur le côté gauche et l'intersection dans la direction haut-bas, et

évaluer l'équilibre entre droite et gauche pendant le déplacement en utilisant au moins l'un parmi le rapport de surface, le rapport de longueur, et le rapport de quantité de réduction.

2. Dispositif d'évaluation selon la revendication 1, dans lequel l'unité d'évaluation est adaptée pour calculer le rapport de surface, le rapport de longueur et le rapport de quantité de réduction pour chaque pas, et évaluer l'équilibre entre droite et gauche pendant le déplacement en utilisant tous les rapports.

3. Dispositif d'évaluation selon la revendication 2, dans lequel l'unité d'évaluation est adaptée pour évaluer si l'équilibre est imparfait du côté gauche ou du côté droit pour chaque pas par rapport à chacun du rapport de surface, du rapport de longueur et du rapport de quantité de réduction, et pour évaluer l'équilibre entre droite et gauche selon une majorité d'un résultat d'évaluation de l'équilibre du côté gauche et du côté droit.

4. Dispositif d'évaluation selon l'une quelconque des revendications 1 à 3, dans lequel l'unité de sortie est adaptée pour délivrer en sortie l'un parmi un rapport de distribution de l'équilibre entre droite et gauche pour une pluralité de pas et des transitions de résultats d'évaluation de l'équilibre entre droite et gauche pour une pluralité de pas.

5. Dispositif d'évaluation selon l'une quelconque des revendications 1 à 4, comprenant en outre une unité de mise en garde (45) adaptée pour mettre en garde lorsqu'un état dans lequel l'équilibre entre droite et gauche est imparfait d'un côté dans les évaluations d'une pluralité de pas réalisées par l'unité d'évaluation.

6. Dispositif d'évaluation selon l'une quelconque des revendications 1 à 4, comprenant en outre :

   une unité de détermination (43) adaptée pour déterminer une démarche de l'animal sur la base des données de mesure, dans lequel
   l'unité de sortie est adaptée pour délivrer en sortie un résultat d'évaluation pour une démarche prédéterminée.

7. Programme d'évaluation qui amène un ordinateur à exécuter un processus comprenant :

   l'évaluation d'un équilibre entre droite et gauche pendant un déplacement, pour chaque pas sur la base de données de mesure obtenues par un capteur porté par un animal qui se déplace à quatre pattes ; et
   la délivrance en sortie de résultats d'évaluation pour une pluralité de pas, dans lequel l'évaluation inclut
   l'obtention d'une trajectoire d'une position d'un bréchet de l'animal pour chaque pas sur la base des données de mesure en projetant des coordonnées de positions respectives entre un point de départ et un point d'arrivée sur un plan vertical reliant les positions du point de départ et du point d'arrivée,
   **caractérisé par** :

   le calcul de l'un parmi
   un rapport de surface d'un côté gauche et d'un côté droit de la trajectoire par rapport à une ligne centrale verticale qui passe à travers une intersection d'un premier segment linéaire, qui relie un point maximal sur le côté gauche de la trajectoire et un point minimal sur le côté droit de la trajectoire, et un second segment linéaire, qui relie un point maximal sur le côté droit de la trajectoire et un point minimal sur le côté gauche la trajectoire, le point maximal étant un point pour lequel une position d'un point suivant dans une direction haut-bas commence par diminuer, et le point minimal étend un point pour lequel une position d'un point suivant dans la direction haut-bas commence par augmenter,

un rapport de longueur d'une portion gauche et d'une portion droite du premier segment linéaire et du second segment linéaire par rapport à la ligne centrale, la portion gauche étant une somme d'une longueur à partir d'un point maximal sur le côté gauche jusqu'à l'intersection et d'une longueur à partir d'un point minimal sur le côté gauche à l'intersection, et la portion droite étant une somme d'une longueur à partir d'un point maximal sur le côté droit jusqu'à l'intersection et d'une longueur à partir d'un point minimal sur le côté droit jusqu'à l'intersection, et

un rapport de quantité de réduction qui est un rapport entre une quantité de réduction droite qui est une différence entre le point minimal sur le côté droit et l'intersection dans la direction haut-bas et une quantité de réduction gauche qui est une différence entre le point minimal sur le côté gauche et l'intersection dans la direction haut-bas, et

l'évaluation de l'équilibre entre droite et gauche pendant le déplacement en utilisant au moins l'un parmi le rapport de surface, le rapport de longueur, et le rapport de quantité de réduction.

# FIG.1

10

EVALUATION
DEVICE
12

MEASUREMENT
DATA
24

MEASURING
DEVICE
11

# FIG.2

WORN

TRAINING (COLLECT DATA)

ANALYZE DATA

AT OFFICE

MEASURING
DEVICE

# FIG.3

```
                                                                    ⌐11
┌─────────────────────────────────────────────────────────────────────┐
│ MEASURING DEVICE                                                      │
│             ⌐20                    ⌐23                    ⌐22         │
│  ┌──────────────┐        ┌──────────────────┐   ┌──────────────────┐  │
│  │ EXTERNAL I/F │        │                  │   │  STORAGE UNIT    │  │
│  │ UNIT         │────────│                  │   │          ⌐24     │  │
│  └──────────────┘        │                  │   │  ┌────────────┐  │  │
│             ⌐21          │  CONTROL UNIT    │───│  │ MEASURE-   │  │  │
│  ┌──────────────┐        │                  │   │  │ MENT DATA  │  │  │
│  │ SENSOR UNIT  │────────│                  │   │  └────────────┘  │  │
│  └──────────────┘        └──────────────────┘   └──────────────────┘  │
└─────────────────────────────────────────────────────────────────────┘
```

# FIG.4

```
                                                                    ⌐12
┌─────────────────────────────────────────────────────────────────────────┐
│ EVALUATION DEVICE                                                         │
│                                    ⌐34                    ⌐33             │
│                         ┌──────────────────┐   ┌──────────────────┐       │
│                         │ CONTROL UNIT     │   │  STORAGE UNIT    │       │
│             ⌐30         │          ⌐40     │   │          ⌐35     │       │
│  ┌──────────────┐       │  ┌────────────┐  │   │  ┌────────────┐  │       │
│  │ EXTERNAL I/F │───────│  │ STORAGE    │  │   │  │ MEASURE-   │  │       │
│  │ UNIT         │       │  │ UNIT       │  │   │  │ MENT DATA  │  │       │
│  └──────────────┘       │  └────────────┘  │   │  └────────────┘  │       │
│                         │          ⌐41     │   │          ⌐36     │       │
│                         │  ┌────────────┐  │   │  ┌────────────┐  │       │
│                         │  │ ACCEPTING  │  │   │  │ EVALUATION │  │       │
│                         │  │ UNIT       │  │   │  │ DATA       │  │       │
│             ⌐31         │  └────────────┘  │   │  └────────────┘  │       │
│  ┌──────────────┐       │          ⌐42     │   │                  │       │
│  │ DISPLAY UNIT │───────│  ┌────────────┐  │───│                  │       │
│  └──────────────┘       │  │ EVALUATING │  │   │                  │       │
│                         │  │ UNIT       │  │   │                  │       │
│                         │  └────────────┘  │   │                  │       │
│                         │          ⌐43     │   │                  │       │
│                         │  ┌────────────┐  │   │                  │       │
│                         │  │ DETERMINING│  │   │                  │       │
│                         │  │ UNIT       │  │   │                  │       │
│             ⌐32         │  └────────────┘  │   │                  │       │
│  ┌──────────────┐       │          ⌐44     │   │                  │       │
│  │ INPUT UNIT   │───────│  ┌────────────┐  │   │                  │       │
│  └──────────────┘       │  │ OUTPUT UNIT│  │   │                  │       │
│                         │  └────────────┘  │   │                  │       │
│                         └──────────────────┘   └──────────────────┘       │
└───────────────────────────────────────────────────────────────────────────┘
```

# FIG.5

MOVEMENT OF
TWO STRIDES

END
POINT

START
POINT

# FIG.6

END
POINT

START
POINT

# FIG.7

# FIG.8

# FIG.9

# FIG.10

# FIG.11A

| INDEX 1 | INDEX 2 | INDEX 3 |
|---------|---------|---------|
| 0.32153 | 1.699408 | 7.064767 |

# FIG.11B

| INDEX 1 | INDEX 2 | INDEX 3 |
|---------|---------|---------|
| 1.052091 | 0.971146 | 1.811758 |

# FIG.11C

| INDEX 1 | INDEX 2 | INDEX 3 |
|---------|---------|---------|
| 21.53606 | 2.837539 | 0.089882 |

# FIG.12A

NORMAL HORSE

20140519

20.86%  23.02%
11.51%  15.11%

MEANINGS OF COLORS OF PIE CHART

ALL THREE INDICES INDICATE LEFT
TWO OF THREE INDICES INDICATE LEFT
ANALYSIS IS IMPOSSIBLE
TWO OF THREE INDICES INDICATE RIGHT
ALL THREE INDICES INDICATE RIGHT

# FIG.12B

HORSE WITH ABNORMAL LEG

20140603

9.95%
9.49%
43.98%
19.21%

20141224

14.12%
33.90%
15.25%
16.95%

MEANINGS OF COLORS OF PIE CHART

ALL THREE INDICES INDICATE LEFT
TWO OF THREE INDICES INDICATE LEFT
ANALYSIS IS IMPOSSIBLE
TWO OF THREE INDICES INDICATE RIGHT
ALL THREE INDICES INDICATE RIGHT

# FIG.13

EP 3 111 841 B1

ALL THREE INDICES INDICATE LEFT
TWO OF THREE INDICES INDICATE LEFT
ANALYSIS IS IMPOSSIBLE
TWO OF THREE INDICES INDICATE RIGHT
ALL THREE INDICES INDICATE RIGHT

T1

FAST WALKING FOR 5 MINUTES (395 STRIDES)

# FIG.14

```
                    ┌─────────────┐
                    │    START    │
                    └─────────────┘
                           │
                           ▼
        ┌──────────────────────────────────────┐
        │  OBTAIN TRAJECTORY OF POSITION OF     │∼ S10
        │           BRISKET OF HORSE            │
        └──────────────────────────────────────┘
                           │
                           ▼
        ┌──────────────────────────────────────┐
        │  EVALUATE BALANCE BETWEEN RIGHT       │∼ S11
        │      AND LEFT FOR EACH STRIDE         │
        └──────────────────────────────────────┘
                           │
                           ▼
        ┌──────────────────────────────────────┐
        │           DETERMINE GAIT             │∼ S12
        └──────────────────────────────────────┘
                           │
                           ▼
        ┌──────────────────────────────────────┐
        │   STORE EVALUATION RESULTS AND        │∼ S13
        │              GAIT                     │
        └──────────────────────────────────────┘
                           │
                           ▼
        ┌──────────────────────────────────────┐
        │      OUTPUT EVALUATION RESULTS        │∼ S14
        └──────────────────────────────────────┘
                           │
                           ▼
                    ┌─────────────┐
                    │     END     │
                    └─────────────┘
```

# FIG.15

EVALUATION DEVICE — 12

CONTROL UNIT — 34

STORAGE UNIT — 33

EXTERNAL I/F UNIT — 30

STORAGE UNIT — 40

MEASURE-MENT DATA — 35

ACCEPTING UNIT — 41

EVALUATION DATA — 36

DISPLAY UNIT — 31

EVALUATING UNIT — 42

DETERMINING UNIT — 43

INPUT UNIT — 32

OUTPUT UNIT — 44

NOTIFYING UNIT — 37

WARNING UNIT — 45

# FIG.16

WORN

13
13A

TRAINING (COLLECT DATA)

12
13
11

ANALYZE DATA

14

12

12

APPLICATION SOFTWARE

EP 3 111 841 B1

# FIG.17

WORN

13
13A

ANALYZE DATA

15

TRAINING (COLLECT DATA)

LTE/3G

13

12

11

12

APPLICATION SOFTWARE

# FIG.18

CORRECT

G

# FIG.19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005115242 A
- JP 2006009959 A
- JP 2008264114 A
- JP 2006218122 A
- JP 2015084943 A